(19) Europäisches Patentamt / European Patent Office / Office européen des brevets

(11) **EP 2 684 035 B1**

(12) **EUROPÄISCHE PATENTSCHRIFT**

(45) Veröffentlichungstag und Bekanntmachung des Hinweises auf die Patenterteilung:
**12.09.2018  Patentblatt 2018/37**

(21) Anmeldenummer: **11799227.1**

(22) Anmeldetag: **14.12.2011**

(51) Int Cl.:
**G01N 27/22** *(2006.01)*    **G01N 33/36** *(2006.01)*

(86) Internationale Anmeldenummer:
**PCT/CH2011/000297**

(87) Internationale Veröffentlichungsnummer:
**WO 2012/122661 (20.09.2012 Gazette 2012/38)**

(54) **VORRICHTUNG UND VERFAHREN ZUR KAPAZITIVEN UNTERSUCHUNG EINES BEWEGTEN PRÜFGUTES**

DEVICE AND METHOD FOR CAPACITIVE ANALYSIS OF A MOVING TEST MATERIAL

PROCÉDÉ ET DISPOSITIF D'INVESTIGATION CAPACITIVE D'UN PRODUIT À CONTRÔLER EN MOUVEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.03.2011  CH 418112011**

(43) Veröffentlichungstag der Anmeldung:
**15.01.2014  Patentblatt 2014/03**

(73) Patentinhaber: **Uster Technologies AG
8610 Uster (CH)**

(72) Erfinder:
• **GEHRIG, Reto
CH-9113 Degersheim (CH)**
• **BLEISCH, Karl
CH-8610 Uster (CH)**
• **KOLLER, Beat
CH-8610 Uster (CH)**

(74) Vertreter: **Pliska, Pavel
Uster Technologies AG
Sonnenbergstrasse 10
8610 Uster (CH)**

(56) Entgegenhaltungen:
EP-A1- 0 924 513    EP-A1- 0 924 518
WO-A1-2005/033697    WO-A1-2008/128363
WO-A1-2010/043063    US-A- 3 731 069
US-B1- 6 369 588

**Beschreibung**

FACHGEBIET

Die vorliegende Erfindung betrifft eine Vorrichtung und ein Verfahren zur kapazitiven Untersuchung eines bewegten strangförmigen, vorzugsweise textilen Prüfgutes gemäss dem Oberbegriff der unabhängigen Patentansprüche. Sie kommt vorzugsweise, aber nicht ausschliesslich, bei der Offline-Messung der Massenungleichmässigkeit von Garn, Vorgarn oder Faserband zum Einsatz, wie sie auf textilen Laborprüfgeräten vorgenommen wird.

STAND DER TECHNIK

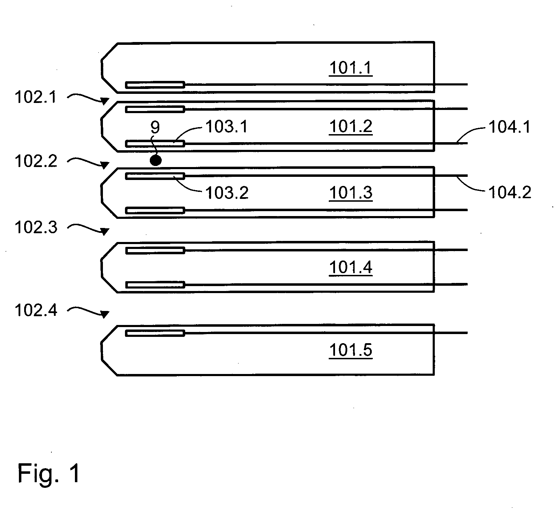

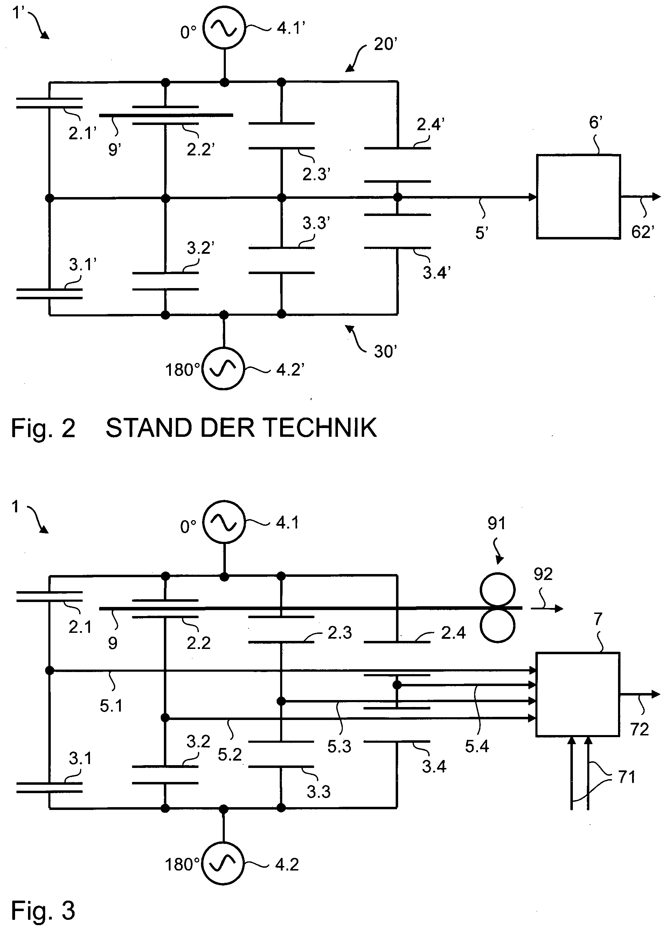

Die EP-0'924'518 A1 offenbart eine Vorrichtung zum kapazitiven Messen von Eigenschaften eines textilen Produktes wie Faserband, Vorgarn oder Garn. Zum besseren Verständnis dieses Stands der Technik werden in den beiliegenden Figuren 1 und 2 einige mit der vorliegenden Erfindung zusammenhängende Aspekte der EP-0'924'518 A1 illustriert.

Die Darstellung von **Figur 1** entspricht derjenigen der Figur 13 der EP-0'924'518 A1 und zeigt fünf Trägerplatten 101.1-101.5, die vier Durchgangsöffnungen oder Messspalte 102.1-102.4 unterschiedlicher Spaltbreiten bilden. Das Prüfgut 9 wird durch einen der Messspalte 102.2 geführt. Da die Trägerplatten 101.1-101.5 im Wesentlichen parallel nebeneinander liegen, kann das Prüfgut 9 nur in genau einen Messspalt 102.2 eingeführt und durch diese entlang seiner Längsachse bewegt werden kann. Die Messspalte 102.1-102.4 weisen jeweils in oder auf ihren beiden Seitenwänden, zwischen denen das Produkt 9 führbar ist, je eine Elektrode 103.1, 103.2 eines Messkondensatorsund je eine (in Figur 1 nicht dargestellte) Elektrode eines Kompensationskondensators auf. Mit dem Kompensationskondensator, dessen Kapazität gleich gross ist wie diejenige des Messkondensators, lassen sich Störeinflüsse wie lokale Feuchtigkeitsänderungen oder Verformungen der Kondensatorgeometrie wirksam kompensieren. Elektrische Leitungen 104.1, 104.2 verbinden die Mess- und Kompensationskondensatoren mit einer (in Figur 1 nicht eingezeichneten) Messschaltung. Die Messspalte 102.1-102.4 weisen unterschiedliche Breiten auf, so dass das Prüfgut 9 je nach seinem Querschnitt in einem Messspalt 102.2 mit geeigneter Breite ausgemessen werden kann. Grundsätzlich ist man bestrebt, die Messspaltbreite so zu wählen, dass einerseits das Prüfgut 9 durch den Messspalt 102.2 geführt werden kann, ohne seine Wände zu berühren, dass aber andererseits der Messspalt 102.2 nicht viel breiter als der Querschnitt des Prüfgutes 9 ist. Je grösser nämlich der vom Prüfgut 9 ausgefüllte Anteil des Messspaltes 102.2 ist, umso höher ist die Empfindlichkeit der Vorrichtung bezüglich sich während der Messung ändernder Eigenschaften, bspw. Massenungleichmässigkeiten, des Prüfgutes 9. Die Breiten der Messspalte 102.1-102.4 können z. B. im Bereich zwischen 0.1 mm und 10 mm liegen.

**Figur 2,** die im Wesentlichen der Figur 2 der EP-0'924'518 A1 entspricht, zeigt eine als Halbmessbrücke ausgebildete Messschaltung 1' mit vier Messkondensatoren 2.1'-2.4' und vier ihnen zugeordneten Kompensationskondensatoren 3.1'-3.4'. Die Messkondensatoren 2.1'-2.4' sind zueinander parallel geschaltet und bilden einen ersten Halbbrückenzweig 20', während die ebenfalls zueinander parallel geschalteten Kompensationskondensatoren 3.1'-3.4' einen zweiten Halbbrückenzweig 30' bilden. Die beiden Halbbrückenzweige 20' 30' sind in Serie zueinander geschaltet, und zwischen ihnen wird auf einer Leitung 5' ein Ausgangssignal abgegriffen. An die beiden Halbbrückenzweige 20', 30' werden von Wechselspannungsgeneratoren 4.1', 4.2' gleich grosse, aber gegenphasige Wechselspannungen angelegt. Wenn die Halbmessbrücke 1' abgeglichen ist und sich kein Prüfgut 9' in einem der Messkondensatoren 2.1'-2.4' befindet, haben die beiden Halbbrückenzweige 20', 30' gleich grosse Gesamtkapazitäten; das Ausgangssignal ist somit null. Wird hingegen ein Prüfgut 9' in einen Messspalt eines Messkondensators 2.2' eingeführt, so beeinflusst es die Kapazität des Messkondensators 2.2'. Die so erzeugte Kapazitätsänderung des betreffenden Messkondensators 2.2' stört das Gleichgewicht zwischen den Messkondensatoren 2.1'-2.4' und den Kompensationskondensatoren 3.1'-3.4', so dass als Ausgangssignal eine Wechselspannung resultiert, deren Amplitude proportional zur Masse des Prüfgutes 9' zwischen den Messelektroden ist. Dieses Ausgangssignal wird in einer Signalverarbeitungseinheit 6' verarbeitet, bspw. verstärkt, gefiltert und/oder gewandelt, und als Mass für die Masse pro Längeneinheit des Prüfgutes 9' ausgewertet.

Aus der WO-2005/033697 A1 oder der US-3,731,069 A sind Vorrichtungen bekannt, die mehrere identische Durchgangsöffnungen mit identischen Messkondensatoren aufweisen. In den entsprechenden Messverfahren werden gleichzeitig mehrere Garne geprüft, nämlich ein Garn in jeder Durchgangsöffnung. Dabei werden die kapazitiven Messsignale der Messkondensatoren seriell oder sequenziell einer gemeinsamen Auswertelektronik zugeführt. Die US-6,369,588 B1 offenbart eine Vorrichtung und ein Verfahren zur kapazitiven Prüfung eines Gewebes. Das entlang seiner Längsrichtung bewegte Gewebe wird auf seiner ganzen Breite von mehreren kapazitiven Sensoren abgetastet. Die Signale der kapazitiven Sensoren werden seriell einem gemeinsamen Mikroprozessor zur Auswertung zugeführt.

In der WO-2008/128363 A1 ist ein Messkondensator für Garn offenbart, welcher zwei in Bewegungsrichtung des Garns hintereinander angeordnete Messteilelektroden aufweist. Die effektive Messfeldlänge kann variiert werden, indem kurze Garnfehler mit nur einer der beiden Messteilelektroden detektiert werden und lange Garnfehler mit beiden zusammen. Die beiden Messteilelektroden können auch zur Geschwindigkeits- oder Längenmessung mittels Laufzeitkorrelation verwendet werden.

DARSTELLUNG DER ERFINDUNG

Es ist Aufgabe der vorliegenden Erfindung, die Vorrichtung und das Messverfahren gemäss der EP-0'924'518 A1 zu verbessern. Insbesondere soll die Messempfindlichkeit erhöht und die Lastkapazität vermindert werden. Die Anzahl der Durchgangsöffnungen soll reduziert und gleichzeitig die Möglichkeit zum Ausmessen sehr dünner Prüfgüter geboten werden. Das Signal-zu-Rauschverhältnis soll erhöht werden. Die Betriebsspannung der Messschaltung soll herabgesetzt und damit die verbrauchte elektrische Leistung reduziert werden. Die Herstellungskosten sollen durch den Einsatz kostengünstigerer Bauteile gesenkt werden. Die Auswirkungen der Verstaubung der Durchgangsöffnungen sollen vermindert werden.

Diese und andere Aufgaben werden durch die erfindungsgemässe Vorrichtung und das erfindungsgemässe Verfahren, wie sie in den unabhängigen Patentansprüchen definiert sind, gelöst. Vorteilhafte Ausführungsformen sind in den abhängigen Patentansprüchen angegeben.

Die Erfindung beruht auf der Idee, bei der Messung nur denjenigen Messkondensator zu berücksichtigen, in dem sich gerade das Prüfgut befindet, während alle anderen Messkondensatoren unberücksichtigt bleiben. Dadurch wird die Gesamtkapazität der Messschaltung reduziert und ihre Empfindlichkeit erhöht. Berechnungen zeigen, dass bei einer Anzahl von $n$ Messkondensatoren (wobei $n \geq 2$ eine natürliche Zahl ist) und unter der Annahme, dass alle Kondensatoren dieselbe Kapazität haben, die Empfindlichkeit der erfindungsgemässen Messschaltung gegenüber der bekannten Messschaltung theoretisch um den Faktor $n$ erhöht werden kann. Dies stellt eine ganz erhebliche Verbesserung dar. Der Vorteil der höheren Empfindlichkeit wird durch den Nachteil eines etwas komplexeren Aufbaus der Messschaltung erkauft. Für die Auswahl des zu berücksichtigenden Messkondensators werden nämlich elektrisch betätigbare Auswahlmittel, z. B. ein Multiplexer oder mehrere Schalter, benötigt.

Die erfindungsgemässe Vorrichtung zur kapazitiven Untersuchung eines bewegten strangförmigen Prüfgutes weist mindestens zwei Durchgangsöffnungen mit unterschiedlichen geometrischen Massen, die derart gegenseitig angeordnet sind, dass das Prüfgut jeweils durch genau eine der mindestens zwei Durchgangsöffnungen entlang seiner Längsachse bewegbar ist, auf. Ferner weist die Vorrichtung einer Fördereinrichtung zur Förderung genau eines Prüfgutes durch genau eine ausgewählte der mindestens zwei Durchgangsöffnungen auf. Schliesslich ist die Vorrichtung mit einer kapazitiven Messschaltung ausgestattet. Die Messschaltung beinhaltet mindestens zwei Messkondensatoren, von denen jeder zur Aufnahme des Prüfgutes ausgelegt ist, wobei jeder der mindestens zwei Messkondensatoren einer der mindestens zwei Durchgangsöffnungen derart zugeordnet ist, dass seine Kapazität von einem in der betreffenden Durchgangsöffnung befindlichen Prüfgut beeinflussbar ist. Die Messschaltung beinhaltet auch Generatormittel, die dazu eingerichtet sind, mindestens ein elektrisches Wechselsignal an die mindestens zwei Messkondensatoren anzulegen. Die Messschaltung beinhaltet ausserdem auch mindestens eine Leitung zur Ausgabe von Ausgangssignalen zumindest eines der Messkondensatoren. Die kapazitive Messschaltung weist elektrisch betätigbare Auswahlmittel auf, mittels derer eine echte Teilmenge der Menge aller Messkondensatoren derart auswählbar ist, dass nur die ausgewählte Teilmenge zur Messung beiträgt. Die zur ausgewählten Teilmenge komplementäre Menge soll also verworfen werden, so dass sie nicht zur Messung beiträgt.

Die ausgewählte Teilmenge von Messkondensatoren beinhaltet vorzugsweise genau einen Messkondensator, nämlich denjenigen, in den das Prüfgut eingeführt ist. Die in dieser Schrift verwendeten Ausdrücke "echte Teilmenge" und "komplementäre Menge" sind durchaus im Sinn der Cantorschen Mengenlehre zu verstehen: Eine Menge B ist eine echte Teilmenge einer Obermenge A ($B \subset A$), wenn jedes Element von B auch in A enthalten ist, und wenn A zudem weitere Elemente enthält, die nicht in B enthalten sind. Die zu B komplementäre Menge ($A \backslash B$) enthält diejenigen Elemente von A, die nicht in B enthalten sind. Zudem soll die ausgewählte Teilmenge im Sinn der vorliegenden Erfindung nicht leer sein, d. h. mindestens einen der Messkondensatoren enthalten.

In einer bevorzugten Ausführungsform sind die mindestens zwei Messkondensatoren parallel zueinander geschaltet. Jedem Messkondensator ist ein Kompensationskondensator zugeordnet. Die Kompensationskondensatoren sind zur Kompensation von die Messung störenden Einflüssen wie lokalen Feuchtigkeitsänderungen oder Verformungen der Kondensatorgeometrie bestimmt, nicht aber zur Aufnahme von Prüfgut. Die Kapazität eines jeden Kompensationskondensators soll gleich gross sein wie diejenige des Messkondensators, dem er zugeordnet ist. Jeder Messkondensator und der ihm zugeordnete Kompensationskondensator bilden vorzugsweise ein in Serie geschaltetes Kondensatorpaar. Wenn der Messkondensator und der ihm zugeordnete Kompensationskondensator in Serie geschaltet sind, greift die Leitung die Ausgangssignale vorzugsweise zwischen dem Messkondensator und dem ihm zugeordneten Kompensationskondensator ab.

Was die Ausgestaltung der Auswahlmittel in der erfindungsgemässen Messschaltung betrifft, so werden die folgenden zwei Ausführungsformen bevorzugt, die auf gewisse Weise komplementär zueinander sind:

(a) Gemäss einer ersten Ausführungsform sind die Auswahlmittel als Schalter, mittels derer eine elektrische Ver-

binding zwischen dem mindestens einen Wechselsignalgenerator und jedem der Messkondensatoren individuell herstellbar und unterbrechbar ist, ausgebildet. Jedem Messkondensator ist mindestens ein Schalter zugeordnet, und alle Messkondensatoren sind mit einer gemeinsamen Leitung zur Ausgabe der Ausgangssignale verbunden. (b) Gemäss einer zweiten Ausführungsform sind die Auswahlmittel als Multiplexer, mittels dessen ein nur von der ausgewählten Teilmenge der Messkondensatoren stammendes Ausgangssignal auswählbar ist, ausgebildet. Alle Messkondensatoren sind mit dem mindestens einen elektrischen Wechselsignalgenerator verbunden, und jedem Messkondensator ist eine individuelle Leitung zur Ausgabe der jeweiligen Ausgangssignale zugeordnet.

Diese Ausführungsformen können trotz ihrer Komplementarität bei Bedarf miteinander kombiniert werden.

Das erfindungsgemässe Verfahren dient zur kapazitiven Untersuchung eines strangförmigen Prüfgutes in einer erfindungsgemässen Vorrichtung nach einem der vorangehenden Ansprüche. Dabei wird das Prüfgut in genau eine der mindestens zwei Durchgangsöffnungen eingeführt und durch diese entlang seiner Längsachse bewegt wird, während sich in den übrigen Durchgangsöffnungen kein Prüfgut befindet. Das Prüfgut beeinflusst eine Kapazität eines der betreffenden Durchgangsöffnung zugeordneten Messkondensators. Mindestens ein elektrisches Wechselsignal wird an den der betreffenden Durchgangsöffnung zugeordneten Messkondensator von den Generatormitteln angelegt. Ausgangssignale zumindest eines der Messkondensatoren werden ausgegeben. Eine echte Teilmenge der Menge aller Messkondensatoren wird mittels der elektrisch betätigbaren Auswahlmittel derart ausgewählt, dass nur die ausgewählte Teilmenge zur Messung beiträgt. Die zur ausgewählten Teilmenge komplementäre Menge wird also verworfen, so dass sie nicht zur Messung beiträgt.

In einer bevorzugten Ausführungsform werden die Messung störende Einflüsse durch Kompensationskondensatoren, von denen jeder einem Messkondensator zugeordnet ist, kompensiert.

Auch das erfindungsgemässe Verfahren hat zwei bevorzugte Ausführungsformen für die Auswahl, die im Wesentlichen denjenigen der erfindungsgemässen Messschaltung entsprechen:

(a) Das mindestens eine elektrische Wechselsignal wird nur an die ausgewählte Teilmenge angelegt, während es von der zur ausgewählten Teilmenge komplementären Menge abgeschaltet wird. Es werden Ausgangssignale aller Messkondensatoren gemeinsam, d. h. auf einer einzigen gemeinsamen Leitung ausgegeben.

(b) Nur Ausgangssignale der ausgewählten Teilmenge werden ausgegeben, während Ausgangssignale der zur ausgewählten Teilmenge komplementären Menge verworfen werden. Das mindestens eine elektrische Wechselsignal wird an alle Messkondensatoren angelegt.

Auch hier ist eine Kombination der beiden Ausführungsformen möglich.

Nachfolgend sind einige Unterschiede zwischen der Erfindung und der Lehre der WO-2005/033697 A1 und der US-3,731,069 A aufgelistet:

- Die WO-2005/033697 A1 und die US-3,731,069 A offenbaren identische Durchgangsöffnungen. Gemäss der Erfindung haben die Durchgangsöffnungen hingegen unterschiedliche geometrische Masse für unterschiedliche Prüfgutdurchmesser.

- Gemäss der WO-2005/033697 A1 und der US-3,731,069 A werden gleichzeitig Messungen an mehreren Garnen vorgenommen, von denen jedes durch eine eigene Durchgangsöffnung bewegt wird. Gemäss der Erfindung soll hingegen nur genau ein Prüfgut auf einmal geprüft werden. Mehrere Durchgangsöffnungen werden bloss deshalb zur Verfügung gestellt, um einen möglichst grossen Durchmesserbereich der Prüfgüter abzudecken.

- Gemäss der WO-2005/033697 A1 und der US-3,731,069 A werden die kapazitiven Messsignale seriell oder sequenziell einer gemeinsamen Auswertelektronik zugeführt. Diese Sequenzen werden möglichst schnell (oftmals pro Sekunde) wiederholt, um möglichst viele Messdaten von allen Garnen zu erhalten. Gemäss der Erfindung findet hingegen keine sequenzielle Auslesung von Messsignalen der einzelnen Messkondensatoren statt. Die erfindungsgemässe Auswahl der Teilmenge der Messkondensatoren ist statisch in dem Sinn, dass eine einmal getroffene Auswahl während der Ausmessung eines Prüfgutes unverändert bleibt. Ein solches Prüfgut sollte mindestens 10 m lang sein, und die Messdauer am selben Prüfgut beträgt z. B. mindestens 10 s.

Die Erfindung bringt eine Reihe von Vorteilen gegenüber der EP-0'924'518 A1 mit sich, von denen einige nachfolgend aufgezählt werden:

- Verbesserung der Empfindlichkeit. Die durch das Prüfgut verursachte Kapazitätsänderung des Messkondensators fliesst direkt in das Ausgangssignal der Messschaltung ein und wird nicht wie bis anhin durch die übrigen, parallel geschalteten Messschlitze abgeschwächt. Bei $n$ Messkondensatoren gleicher Kapazität erhöht sich die Empfindlichkeit theoretisch um den Faktor $n$.

- Erhöhung des Signal-zu-Rauschverhältnisses. Wie oben erklärt, erhöht sich die Empfindlichkeit. Das Rauschen der Messschaltung bleibt jedoch gleich. Somit resultiert eine Erhöhung des Signal-zu-Rauschverhältnisses um den Faktor *n* (bei *n* Messkondensatoren gleicher Kapazität).

- Ausmessen sehr dünner textiler Prüfgüter. Die höhere Empfindlichkeit lässt sich z. B. dazu verwenden, sehr feine Garne auszumessen. Dieser Aspekt könnte besonders bei Chemiefasern interessant sein.
- Reduktion der Anzahl Messschlitze. Während bisher ein sehr dünnes textiles Prüfgut in einem Messschlitz mit sehr kleinem Elektrodenabstand ausgemessen werden musste, kann dasselbe Prüfgut dank der vorliegenden Erfindung in einem breiteren Messschlitz ausgemessen werden. Dank der höheren Empfindlichkeit kann auch das Signal aus dem breiteren Messschlitz noch genügend sein.
- Herabsetzung der Generatorspannung. Die höhere Empfindlichkeit kann es auch erlauben, die Messschaltung mit einer niedrigeren Generatorspannung zu betreiben. Dies führt zu einer Reduktion der verbrauchten Leistung und somit zu einer geringeren Erhitzung. Ausserdem stehen für die Ausführung der Messschaltung mehr Bauteile zur Verfügung, die den Anforderungen der Endstufe genügen und preisgünstiger sind.
- Verminderung der Lastkapazität. Die vorliegende Erfindung senkt die Gesamtkapazität der Messschaltung. Dadurch hat die aktive Endstufe weniger Last, wird noch weniger erhitzt und neigt weniger zu störenden Schwingungen.
- Entschärfung der Verstaubungsproblematik. Besonders bei hohen Abzugsgeschwindigkeiten können gewisse Garne viel Staub entwickeln. Dieser lagert sich im momentan verwendeten Messschlitz, aber auch in den übrigen Messschlitzen ab, was zu einer Drift des Ausgangssignals führt. Diese Drift wird mit der vorliegenden Erfindung dadurch zumindest abgeschwächt, dass die nicht momentan verwendeten Messschlitze keinen Einfluss mehr auf das Ausgangssignal haben.

## AUFZÄHLUNG DER ZEICHNUNGEN

Nachfolgend werden bevorzugte Ausführungsformen der Erfindung anhand der Zeichnungen detailliert erläutert. Zum Vergleich ist in Figur 2 auch Stand der Technik dargestellt.

Figur 1          zeigt Teile einer erfindungsgemässen Vorrichtung, die auch aus der EP-0'924'518 A1 bekannt sind.
Figur 2          zeigt eine aus der EP-0'924'518 Albekannte kapazitive Messschaltung.
Figuren 3-5     zeigen drei Ausführungsformen einer erfindungsgemässen Messschaltung.

## AUSFÜHRUNG DER ERFINDUNG

**Figur 3** zeigt ein Schaltschema einer ersten Ausführungsform der erfindungsgemässen Messschaltung 1. Diese Messschaltung 1 hat z. B. vier Messkondensatoren 2.1-2.4, die als ebene Plattenkondensatoren ausgebildet sein können. Jeder der Messkondensatoren 2.1-2.4 ist einer Durchgangsöffnung 102.1-102.4 zugeordnet, wie sie etwa in Figur 1 dargestellt sind. Die in Figur 1 eingezeichneten Elektroden 103.1, 103.2 könnten z. B. die Elektroden des Messkondensators 2.2 von Figur 3 sein. Entsprechend den unterschiedlich breiten Durchgangsöffnungen 102.1-102.4 haben auch die ihnen zugeordneten Messkondensatoren 2.1-2.4 unterschiedliche Elektrodenabstände. Wie in Figur 3 angedeutet, sind die Durchgangsöffnungen bzw. die Messkondensatoren 2.1-2.4 derart gegenseitig angeordnet, dass ein textiles Prüfgut 9 jeweils in genau einen Messkondensator 2.2 einführbar ist. Zu diesem Zweck sind die Messkondensatoren 2.1-2.4 in Richtung quer zu ihren Kondensatorplatten gegeneinander versetzt. Eine Versetzung in Richtung parallel zu den Kondensatorplatten ist hingegen nicht nötig und in Figur 3 nur eingezeichnet, um die Elemente der Messschaltung 1 übersichtlicher darstellen zu können.

Das textile Prüfgut 9 wird während der Messung entlang seiner Längsrichtung durch genau einen der Messkondensatoren 2.1-2.4 bewegt; im Beispiel von Figur 3 ist dies der zweite Messkondensator 2.2. Die Bewegung des Prüfgutes 9 wird durch eine Fördereinrichtung 91 erzeugt, und die Bewegungsrichtung ist mit einem Pfeil 92 angedeutet. Die Fördereinrichtung kann z. B. in bekannter Weise als Rollenlieferwerk mit zwei zusammen wirkenden Förderrollen, von denen mindestens eine zur Rotation angetrieben ist, ausgebildet sein. Die Fördereinrichtung 91 ist zur Förderung genau eines Prüfgutes 9 durch genau eine ausgewählte 102.2 aus allen Durchgangsöffnungen 102.1-102.4 eingerichtet.

Die Messschaltung 1 weist ausserdem vier Kompensationskondensatoren 3.1-3.4 auf, die jeweils einem Messkondensator 2.1-2.4 zugeordnet sind und mit diesem ein Kondensatorpaar bilden. Ein Messkondensator 2.1 und der ihm zugeordnete Kompensationskondensator 3.1 sollten möglichst gleiche Kapazitäten aufweisen und sind in Serie geschaltet. Die Kapazitäten der Messkondensatoren 2.1-2.4 können untereinander gleich oder unterschiedlich sein. Die Messkondensatoren 2.1-2.4 sind zur Aufnahme eines länglichen textilen Prüfguts 9, bspw. eines Garns, ausgelegt, die Kompensationskondensatoren 3.1-3.4 dagegen nicht.

Wechselsignalgeneratoren 4.1, 4.2 legen zwei zueinander antisymmetrische elektrische Wechselsignale an die Kondensatorpaare 2.1, 3.1 bis 2.4, 3.4 an. Das heisst, dass die Differenzen der beiden angelegten Wechselsignale zu einem konstanten Referenzsignal betragsmässig gleich und um 180° gegeneinander phasenverschoben sind. Es kann ein einziger Wechselsignalgenerator oder mehrere Wechselsignalgeneratoren 4.1, 4.2 eingesetzt werden. Der mindestens eine Wechselsignalgenerator 4.1, 4.2 kann, wie in der EP-0'924'518 A1 beschrieben, als LC-Oszillator ausgeführt sein, der zusammen mit Elementen 2.1-2.4, 3.1-3.4 der Messschaltung 1 einen elektrischen Schwingkreis bildet. Alternativ können Elemente 2.1-2.4, 3.1-3.4 der Messschaltung 1 vom mindestens einen Wechselsignalgenerator 4.1, 4.2 derart abgekoppelt sein, dass sie Parameter des vom mindestens einen Wechselsignalgenerator 4.1, 4.2 erzeugten elektrischen Wechselsignals nicht wesentlich beeinflussen, wie in der WO-2010/043064 A1 beschrieben. Im letzteren Fall ist der mindestens eine Wechselsignalgenerator 4.1, 4.2 vorzugsweise als Synthesizer ausgeführt, d. h. als eine mixed-signal (digitale und analoge) elektrische Vorrichtung zur Erzeugung von analogen elektrischen Wechselspannungssignalen. Mit besonderem Vorteil wird ein direkter digitaler Synthesizer (direct digital synthesizer, DDS) eingesetzt. So können Parameter des elektrischen Wechselsignals, z. B. seine Grundfrequenz, frei gewählt werden.

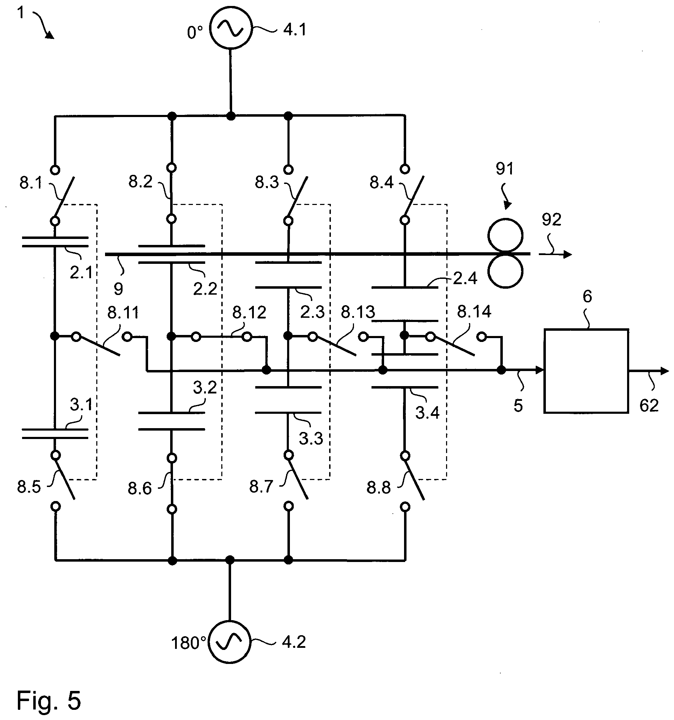

Im Gegensatz zum Stand der Technik gemäss Figur 2 werden in Figur 3 die Ausgangssignale der einzelnen Kondensatorpaare 2.1, 3.1 bis 2.4, 3.4 individuell abgegriffen. Die Abgriffe erfolgen auf Leitungen 5.1-5.4 jeweils zwischen dem Messkondensator 2.1-2.4 und dem ihm zugeordneten Kompensationskondensator 3.1-3.4. Die vier Leitungen 5.1-5.4 münden in einen Multiplexer 7, der aus ihnen eine einzige Leitung 5.2 auswählt und deren Signal auf einer Ausgangsleitung 72 ausgibt. Der Multiplexer 7 kann z. B. als analoger Hochfrequenzmultiplexer ausgeführt sein. Ein oder mehrere Steuersignale 71 geben dem Multiplexer 7 vor, welche der Leitungen 5.1-5.4 bzw. welches Ausgangssignal auszuwählen ist. Die Steuersignale 71 können von einem an sich bekannten, vorzugsweise digitalen (nicht eingezeichneten) Treiberbaustein geliefert werden. Wie aus dem Stand der Technik bekannt, kann das Ausgangssignal vor oder hinter dem Multiplexer 7 verarbeitet, bspw. verstärkt, gefiltert und/oder gewandelt, werden. Statt des Multiplexers 7 könnten einzelne Schalter, welche die Kondensatorpaare 2.1, 3.1 bis 2.4, 3.4 mit einer gemeinsamen Leitung verbinden, vorgesehen sein (vgl. Figur 5).

Die nachfolgende Tabelle vergleicht die Eigenschaften der Schaltungen 1', 1 gemäss den Figuren 2 und 3 miteinander. Dabei werden folgende Annahmen getroffen:

• Die von den Wechselsignalgeneratoren 4.1', 4.2' bzw. 4.1, 4.2 angelegten Wechselspannungen gegenüber Masse betragen $U_{\pm} = \pm \dfrac{1}{2} U_0 \sin \omega t$ , worin $U_0$ eine Spannungsamplitude, $\omega$ eine Kreisfrequenz und $t$ die Zeit ist.

• Das Prüfgut 9' bzw. 9 befindet sich z. B. im zweiten Messkondensator 2.2' bzw. 2.2, welcher die Kapazität $C_2$ habe.

• $U$ ist die abgegriffene Ausgangsspannung gegenüber Masse.

• Alle Kondensatoren 2.1'-2.4', 3.1'-3.4' bzw. 2.1-2.4, 3.1-3.4 haben ohne Prüfgut dieselbe Kapazität $C$.

• Die durch das Prüfgut 9' bzw. 9 hervorgerufene relative Kapazitätsänderung $\left| \dfrac{\Delta C_2}{C_2} \right|$ ist klein, z. B. < 0.1, so dass die

lineare Näherung $\Delta U \approx \dfrac{\partial U}{\partial C_2} \Delta C_2$ verwendet werden kann.

• Parasitäre Kapazitäten werden vernachlässigt.

| | Stand der Technik (Fig. 2) | Erfindung (Fig. 3) |
|---|---|---|
| Gesamtkapazität $C_{\text{tot}}$ | $\dfrac{4C(C_2 + 3C)}{C_2 + 7C}$ | $\dfrac{C_2 C}{C_2 + C}$ |
| $C_{\text{tot}}(C_2=C)$ | 2C | $\dfrac{C}{2}$ |
| Ausgangsspannung $U$ | $\dfrac{U_0}{2} \cdot \dfrac{C_2 - C}{C_2 + 7C}$ | $\dfrac{U_0}{2} \cdot \dfrac{C_2 - C}{C_2 + C}$ |
| $U(C_2=C)$ | 0 | 0 |
| Empfindlichkeit $\dfrac{\partial U}{\partial C_2}$ | $U_0 \dfrac{4C}{(C_2 + 7C)^2}$ | $U_0 \dfrac{C}{(C_2 + C)^2}$ |

(fortgesetzt)

| | Stand der Technik (Fig. 2) | Erfindung (Fig. 3) |
|---|---|---|
| $\left.\dfrac{\partial U}{\partial C_2}\right\|_{C_2=C}$ | $\dfrac{U_0}{16C}$ | $\dfrac{U_0}{4C}$ |

Man sieht, dass die erfindungsgemässe Messschaltung 1 eine viermal kleinere Gesamtkapazität $C_{tot}$ (ohne die Wechselsignalgeneratoren 4.1, 4.2) und eine viermal grössere Empfindlichkeit $\left.\dfrac{\partial U}{\partial C_2}\right\|_{C_2=C}$ hat als die Messschaltung 1' gemäss dem Stand der Technik. Aus dieser Empfindlichkeitssteigerung um den Faktor $n = 4$ ergeben sich die eingangs beschriebenen Vorteile der Erfindung.

Die Auswahl eines einzigen Messkondensators 2.2 stellt übrigens einen Spezialfall derjenigen Situation dar, in welcher eine Anzahl $m$ ($1 \leq m < n$) von Messkondensatoren ausgewählt wird. Im allgemeinen Fall wird die Empfindlichkeit um den Faktor $\dfrac{n}{m}$ erhöht. Diese theoretischen Aussagen wurden zum Teil in Experimenten bereits bestätigt, wobei jedoch die experimentellen Resultate wegen parasitärer Kapazitäten etwas schlechter sind als die theoretischen Voraussagen. Der praktische Nutzen der Erfindung ist jedenfalls nachgewiesen. Die Auswahl von mehr als einem Messkondensator könnte vorteilhaft sein, um eine gezielte Dämpfung des Ausgangssignals zu erzielen, etwa bei einem Prüfgut mit grosser Massendichte oder grosser Dielektrizitätskonstante.

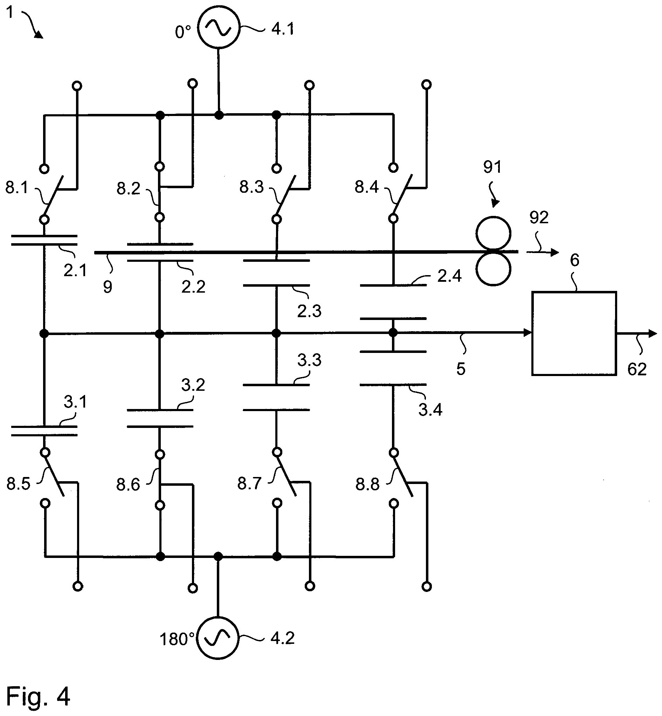

In **Figur 4** ist eine zweite Ausführungsform der erfindungsgemässen Messschaltung 1 dargestellt, die ebenfalls vier Kondensatorpaare 2.1, 3.1 bis 2.4, 3.4 aufweist. Hier können die elektrischen Verbindungen zwischen den Kondensatorpaaren 2.1, 3.1 bis 2.4, 3.4 und den Wechselsignalgeneratoren 4.1, 4.2 einzeln unterbrochen und hergestellt werden. Dies wird mit Schaltern 8.1-8.8 bewerkstelligt, die von entsprechenden, vorzugsweise digitalen Steuersignalen geschaltet werden. Die Schalter 8.1-8.8 können z. B. als Relais oder als analoge Halbleiterschalter ausgebildet sein. Das einem Kondensatorpaar 2.1, 3.1 zugeordnete Schalterpaar 8.1, 8.5 ist vorzugsweise derart gekoppelt, dass es parallel geschaltet wird, d. h. die beiden Schalter 8.1, 8.5 sind entweder beide offen oder beide geschlossen. Die Steuersignale können von einem an sich bekannten, vorzugsweise digitalen (nicht eingezeichneten) Treiberbaustein geliefert werden. Um von der Kompensation zu profitieren und die Messschaltung 1 am Arbeitspunkt $U = 0$ zu betreiben, sollten die einem Kondensatorpaar 2.2, 3.2 zugeordneten Schalter 8.2, 8.6 entweder beide offen oder beide geschlossen sein. Die grössten Vorteile ergeben sich, wenn genau ein Kondensatorpaar 2.2, 3.2, z. B. das zweite Kondensatorpaar, an den Wechselsignalgeneratoren 4.1, 4.2 angeschlossen und alle anderen Kondensatorpaare 2.1, 3.1; 2.3, 3.3; 2.4, 3.4 von den Wechselsignalgeneratoren 4.1, 4.2 getrennt sind. In dieser zweiten Ausführungsform kann eine einzige Leitung 5 alle Kondensatorpaare 2.1, 3.1 bis 2.4, 3.4 mit der Signalverarbeitungseinheit 6 verbinden, wie dies beim Stand der Technik der Fall ist. Ein Multiplexer wird hier nicht benötigt.

Zwischen den Ausführungsformen der Figuren 3 und 4 besteht eine Art Komplementarität. In der ersten Ausführungsform bleiben alle Kondensatorpaare 2.1, 3.1 bis 2.4, 3.4 an den Wechselsignalgeneratoren 4.1, 4.2 angeschlossen, dafür wird nur die dem mit Prüfgut 9 beladenen Messkondensator 2.2 zugeordnete Leitung 5.2 für Ausgangssignale zugeschaltet, während die anderen Leitungen 5.1, 5.3, 5.4 abgeschaltet sind. In der zweiten Ausführungsform wird nur dasjenige Kondensatorpaar 2.2, 3.2 and die Wechselsignalgeneratoren 4.1, 4.2 angeschlossen, dessen Messkondensator 2.2 mit Prüfgut 9 beladen ist, während die anderen Kondensatorpaare 2.1, 3.1; 2.3, 3.3; 2.4, 3.4 abgeschaltet sind, dafür bleiben alle Kondensatorpaare 2.1, 3.1 bis 2.4, 3.4 an der gemeinsamen Leitung 5 für Ausgangssignale angeschlossen.

**Figur 5** zeigt eine dritte Ausführungsform der erfindungsgemässen Messschaltung 1. Diese dritte Ausführungsform stellt eine Vereinigung der Ausführungsfonnen der Figuren 3 und 4 dar. Es sind sowohl Schalter 8.11-8.14 zwischen den Kondensatorpaaren 2.1, 3.1 bis 2.4, 3.4 und der gemeinsamen Leitung 5 als auch Schalter 8.1-8-8 zwischen den Wechselsignalgeneratoren 4.1, 4.2 und den Kondensatorpaaren 2.1, 3.1 bis 2.4, 3.4 vorhanden. Das einem Kondensatorpaar 2.1, 3.1 zugeordnete Schaltertripel 8.1, 8.5, 8.11 ist vorzugsweise derart gekoppelt, dass es parallel geschaltet wird, d. h. die drei Schalter 8.1, 8.5, 8.11 sind entweder alle offen oder alle geschlossen. Dies ist in Figur 5 mit gestrichelten Linien angedeutet. Die Schalter 8.1-8.8, 8.11-8.14 können z. B. als Relais oder als analoge Halbleiterschalter ausgebildet sein. Dank der doppelten Abtrennung der nicht ausgewählten Kondensatorpaare 2.1, 3.1; 2.3, 3.3; 2.4, 3.4 werden parasitäre Kopplungen, die Lastkapazität und die Ausgangskapazität reduziert, was besonders vorteilhaft sein kann.

Die erfindungsgemässe Messschaltung 1 kann Abgleichmittel zum Nullabgleich ohne Prüfgut beinhalten. Derartige Abgleichmittel sind z. B. aus den Schriften EP-0'924'518 A1, WO-2010/043063 A1 und WO-2010/043065 A1 bekannt. Die Abgleichmittel sind der Einfachheit halber in den Zeichnungen nicht dargestellt.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

BEZUGSZEICHENLISTE

1    Messschaltung

2.1-2.4    Messkondensatoren
20'    Halbbrückenzweig

3.1-3.4    Kompensationskondensatoren
30'    Halbbrückenzweig

4.1, 4.2    Wechselsignalgeneratoren

5, 5.1-5.4    Leitungen für Ausgangssignale

6    Signalverarbeitungseinheit
62    Ausgangsleitung

7    Multiplexer
71    Steuersignale
72    Ausgangsleitung

8.1-8.8,8.11-8.14    Schalter

9    Prüfgut
91    Fördereinrichtung
92    Bewegungsrichtung des Prüfgutes

101.1-101.5    Trägerplatten
102.1-102.4    Messspalte
103.1, 103.2    Kondensatorelektroden
104.1, 104.2    elektrische Leitungen

**Patentansprüche**

**1.** Vorrichtung zur kapazitiven Untersuchung eines bewegten strangförmigen Prüfgutes (9), mit
mindestens zwei Durchgangsöffnungen (102.1-102.4) mit unterschiedlichen geometrischen Massen, die derart gegenseitig angeordnet sind, dass das Prüfgut (9) jeweils durch genau eine (102.2) der mindestens zwei Durchgangsöffnungen (102.1-102.4) entlang seiner Längsachse bewegbar ist,
einer Fördereinrichtung (91) zur Förderung genau eines Prüfgutes (9) durch genau eine ausgewählte (102.2) der mindestens zwei Durchgangsöffnungen (102.1-102.4),
und
einer kapazitiven Messschaltung (1), die

mindestens zwei Messkondensatoren (2.1-2.4), von denen jeder zur Aufnahme des Prüfgutes (9) ausgelegt ist, wobei jeder der mindestens zwei Messkondensatoren (2.1-2.4) einer der mindestens zwei Durchgangsöffnungen (102.1-102.4) derart zugeordnet ist, dass seine Kapazität ($C_2$) von einem in der betreffenden Durchgangsöffnung (102.2) befindlichen Prüfgut (9) beeinflussbar ist,
Generatormittel (4.1, 4.2), die dazu eingerichtet sind, mindestens ein elektrisches Wechselsignal an die mindestens zwei Messkondensatoren (2.1-2.4) anzulegen, und
mindestens eine Leitung (5, 5.1-5.4) zur Ausgabe von Ausgangssignalen zumindest eines der Messkondensatoren (2.1-2.4)

beinhaltet,
**dadurch gekennzeichnet,**
**dass** die kapazitive Messschaltung (1) elektrisch betätigbare Auswahlmittel (7, 8.1-8.8, 8.11-8.14) aufweist, mittels derer eine echte Teilmenge (2.2) der Menge aller Messkondensatoren (2.1-2.4) derart auswählbar ist, dass nur die ausgewählte Teilmenge (2.2) zur Messung beiträgt.

2. Vorrichtung nach Anspruch 1, wobei jedem Messkondensator (2.1-2.4) ein Kompensationskondensator (3.1-3.4) zur Kompensation von die Messung störenden Einflüssen zugeordnet ist, und jeder Messkondensator (2.2) und der ihm zugeordnete Kompensationskondensator (3.2) ein in Serie geschaltetes Kondensatorpaar (2.2, 3.2) bilden.

3. Vorrichtung nach Anspruch 2, wobei die Leitung (5, 5.2) das Ausgangssignal zwischen dem Messkondensator (2.2) und dem Kompensationskondensator (3.2), die das Kondensatorpaar (2.2, 3.2) bilden, abgreift.

4. Vorrichtung nach einem der vorangehenden Ansprüche, wobei die Auswahlmittel als Schalter (8.1-8.8), mittels derer eine elektrische Verbindung zwischen den Generatormitteln (4.1, 4.2) und jedem der Messkondensatoren (2.1-2.4) individuell herstellbar und unterbrechbar ist, ausgebildet sind.

5. Vorrichtung nach Anspruch 4 und einem der Ansprüche 2 und 3, wobei die Generatormittel (4.1, 4.2) zum Anlegen gleich grosser, aber gegenphasiger elektrischer Wechselsignale an die mindestens zwei Messkondensatoren (2.1-2.4) bzw. an die mindestens zwei Kompensationskondensatoren (3.1-3.4) eingerichtet sind, und die Auswahlmittel sowohl als Schalter (8.1-8.4), mittels derer eine elektrische Verbindung zwischen den Generatormitteln (4.1) und jedem der Messkondensatoren (2.1-2.4) individuell herstellbar und unterbrechbar ist, als auch als Schalter (8.5-8.8), mittels derer eine elektrische Verbindung zwischen den Generatormitteln (4.2) und jedem der Kompensationskondensatoren (3.1-3.4) individuell herstellbar und unterbrechbar ist, ausgebildet sind.

6. Vorrichtung nach Anspruch 4 oder 5, wobei jedem Messkondensator (2.2) mindestens ein Schalter (8.2, 8.6) zugeordnet ist und alle Messkondensatoren (2.1-2.4) mit einer gemeinsamen Leitung (5) zur Ausgabe der Ausgangssignale verbindbar sind.

7. Vorrichtung nach Anspruch 6, wobei die Auswahlmittel als Schalter (8.1-8.8), mittels derer eine elektrische Verbindung zwischen den Generatormitteln (4.1, 4.2) und jedem der Messkondensatoren (2.1-2.4) individuell herstellbar und unterbrechbar ist, und als Schalter (8.11-8.14), mittels derer eine elektrische Verbindung zwischen jedem der Messkondensatoren (2.1-2.4) und der gemeinsamen Leitung (5) individuell herstellbar und unterbrechbar ist, ausgebildet sind.

8. Vorrichtung nach einem der vorangehenden Ansprüche, wobei jedem Messkondensator (2.1-2.4) eine individuelle Leitung (5.1-5.4) zur Ausgabe seiner Ausgangssignale zugeordnet ist und die Auswahlmittel als Multiplexer (7), mittels dessen nur von der ausgewählten Teilmenge (2.2) der Messkondensatoren (2.1-2.4) stammende Ausgangssignale auswählbar sind, ausgebildet sind.

9. Verfahren zur kapazitiven Untersuchung eines strangförmigen Prüfgutes (9) in einer Vorrichtung nach einem der vorangehenden Ansprüche, wobei das Prüfgut (9) in genau eine (102.2) der mindestens zwei Durchgangsöffnungen (102.1-102.4) eingeführt und durch diese entlang seiner Längsachse bewegt wird, während sich in den übrigen Durchgangsöffnungen (102.1, 102.3, 102.4) kein Prüfgut befindet,
das Prüfgut (9) eine Kapazität ($C_2$) eines der betreffenden Durchgangsöffnung (102.2) zugeordneten Messkondensators (2.2) beeinflusst,
mindestens ein elektrisches Wechselsignal an den der betreffenden Durchgangsöffnung (102.2) zugeordneten Messkondensator (2.2) von den Generatormitteln (4.1, 4.2) angelegt wird, und
Ausgangssignale zumindest eines der Messkondensatoren (2.1-2.4) ausgegeben werden,
**dadurch gekennzeichnet, dass**
eine echte Teilmenge (2.2) der Menge aller Messkondensatoren (2.1-2.4) mittels der elektrisch betätigbaren Auswahlmittel (7, 8.1-8.8) derart ausgewählt wird, dass nur die ausgewählte Teilmenge (2.2) zur Messung beiträgt.

10. Verfahren nach Anspruch 9, wobei die Messung störende Einflüsse durch Kompensationskondensatoren (3.1-3.4), von denen jeder einem Messkondensator (2.1-2.4) zugeordnet ist, kompensiert werden.

11. Verfahren nach Anspruch 9 oder 10, wobei zur Auswahl das mindestens eine elektrische Wechselsignal nur an die ausgewählte Teilmenge (2.2) angelegt wird, während es von der zur ausgewählten Teilmenge (2.2) komplementären Menge (2.1, 2.3, 2.4) abgeschaltet wird.

12. Verfahren nach Anspruch 11, wobei Ausgangssignale aller Messkondensatoren (2.1-2.4) gemeinsam ausgegeben werden.

13. Verfahren nach einem der Ansprüche 9-11, wobei nur Ausgangssignale der ausgewählten Teilmenge (2.2) ausgegeben werden, während Ausgangssignale der zur ausgewählten Teilmenge (2.2) komplementären Menge (2.1, 2.3, 2.4) verworfen werden.

14. Verfahren nach den Ansprüchen 11 und 13, wobei zur Auswahl das mindestens eine elektrische Wechselsignal nur an die ausgewählte Teilmenge (2.2) angelegt wird, während es von der zur ausgewählten Teilmenge (2.2) komplementären Menge (2.1, 2.3, 2.4) abgeschaltet wird, und nur Ausgangssignale der ausgewählten Teilmenge (2.2) ausgegeben werden, während Ausgangssignale der zur ausgewählten Teilmenge (2.2) komplementären Menge (2.1, 2.3, 2.4) verworfen werden.

15. Verfahren nach Anspruch 13, wobei das mindestens eine elektrische Wechselsignal an alle Messkondensatoren (2.1-2.4) angelegt und nur Ausgangssignale der ausgewählten Teilmenge (2.2) ausgegeben werden.

## Claims

1. A device for the capacitive analysis of a moved strand-like test material (9), comprising
   at least two lead-through openings (102.1-102.4) with different geometrical masses, which are mutually arranged in such a way that the test material (9) is movable along its longitudinal axis through precisely one (102.2) of the at least two lead-through openings (102.1-102.4),
   a conveying mechanism (91) for conveying precisely one test material (9) through precisely one selected (102.2) of the at least two lead-through openings (102.1-102.4), and
   a capacitive measuring circuit (1) which contains

   at least two measuring capacitors (2.1-2.4), each of which is configured for receiving the test material (9), wherein each of the at least two measuring capacitors (2.1-2.4) is associated with at least one of the at least two lead-through openings (102.1-102.4) in such a way that its capacitance ($C_2$) is influenceable by a test material (9) situated in the respective lead-through opening (102.2),
   generator means (4.1, 4.2) which are configured to apply at least one electrical alternating signal to the at least two measuring capacitors (2.1-2.4), and
   at least one line (5, 5.1-5.4) for the output of output signals of at least one of the measuring capacitors (2.1-2.4),

   **characterized in that**
   the capacitive measuring circuit (1) comprises electrically actuatable selection means (7, 8.1-8.8, 8.11-8.14), by means of which a true subset (2.2) of the set of all measuring capacitors (2.1-2.4) is selectable in such a way that only the selected subset (2.2) contributes to the measurement.

2. The device according to claim 1, wherein each measuring capacitor (2.1-2.4) is associated with a compensation capacitor (3.1-3.4) for compensating influences which disturb the measurement, and wherein each measuring capacitor (2.2) and the compensation capacitor (3.2) associated therewith form a capacitor pair (2.2, 3.2) connected in series.

3. The device according to claim 2, wherein the line (5, 5.2) taps the output signal between the measuring capacitor (2.2) and the compensation capacitor (3.2), which form the capacitor pair (2.2, 3.2).

4. The device according to one of the preceding claims, wherein the selection means are configured as switches (8.1-8.8), by means of which an electrical connection between the generator means (4.1, 4.2) and each of the measuring capacitors (2.1-2.4) can be established and interrupted individually.

5. The device according to claim 4 and one of the claims 2 and 3, wherein
   the generator means (4.1, 4.2) are configured for applying equally large, but antiphase electrical alternating signals to the at least two measuring capacitors (2.1-2.4) and to the at least two compensation capacitors (3.1-3.4), respectively, and

the selection means are configured both as switches (8.1-8.4) by means of which an electric connection between the generator means (4.1) and each of the measuring capacitors (2.1-2.4) can be established and interrupted individually, and as switches (8.5-8.8) by means of which an electric connection between the generator means (4.2) and each of the compensation capacitors (3.1-3.4) can be established and interrupted individually.

6. The device according to claim 4 or 5, wherein at least one switch (8.2, 8.6) is associated with each measuring capacitor (2.2), and all measuring capacitors (2.1-2.4) are connectable to a common line (5) for the output of output signals.

7. The device according to claim 6, wherein the selection means are configured as switches (8.1-8.8) by means of which an electric connection between the generator means (4.1, 4.2) and each of the measuring capacitors (2.1-2.4) can be established and interrupted individually, and as switches (8.11-8.14) by means of which an electric connection between each of the measuring capacitors (2.1-2.4) and the common line (5) can be established and interrupted individually.

8. The device according to one of the preceding claims, wherein each measuring capacitor (2.1-2.4) is associated with an individual line (5.1-5.4) for the output of its output signals, and the selection means are configured as multiplexers (7), by means of which output signals are selectable which only originate from the selected subset (2.2) of the measuring capacitors (2.1-2.4).

9. A method for the capacitive analysis of a strand-like test material (9) in a device according to one of the preceding claims, wherein
the test material (9) is inserted into precisely one (102.2) of the at least two lead-through openings (102.1-102.4) and is moved through said opening along its longitudinal axis, whereas no test material is situated in the remaining lead-through openings (102.1, 102.3, 102.4),
the test material (9) influences a capacitance ($C_2$) of a measuring capacitor (2.2) associated with the respective lead-through opening (102.2),
at least one electrical alternating signal is applied by the generator means (4.1, 4.2) to the measuring capacitor (2.2) associated with the respective lead-through opening (102.2), and
output signals of at least one of the measuring capacitors (2.1-2.4) are output,
**characterized in that**
a true subset (2.2) of the set of all measuring capacitors (2.1-2.4) is selected by means of the electrically actuatable selection means (7, 8.1-8.8) in such a way that only the selected subset (2.2) contributes to the measurement.

10. The method according to claim 9, wherein influences that disturb the measurement are compensated by compensation capacitors (3.1-3.4), each of which is associated to a measuring capacitor (2.1-2.4).

11. The method according to claim 9 or 10, wherein the at least one electrical alternating signal is only applied to the selected subset (2.2), whereas it is deactivated by the set (2.1, 2.3, 2.4) which is complementary to the selected subset (2.2).

12. The method according to claim 11, wherein the output signals of all measuring capacitors (2.1-2.4) are output jointly.

13. The method according to one of the claims 9-11, wherein only output signals of the selected subset (2.2) are output, whereas output signals of the set (2.1, 2.3, 2.4) which is complementary to the selected subset (2.2) are disregarded.

14. The method according to the claims 11 and 13, wherein the at least one electrical alternating signal is applied only to the selected subset (2.2), whereas it is deactivated for the set (2.1, 2.3, 2.4) complementary to the selected subset (2.2), and only output signals of the selected subset (2.2) are output, whereas output signals of the set (2.1, 2.3, 2.4) which is complementary to the selected subset (2.2) are disregarded.

15. The method according to claim 13, wherein the at least one electrical alternating signal is applied to all measuring capacitors (2.1-2.4) and only output signals of the selected subset (2.2) are output.

**Revendications**

1. Dispositif pour l'examen capacitif d'un produit à contrôler en forme de brin (9) en mouvement, avec

au moins deux ouvertures de passage (102.1-102.4) de dimensions géométriques différentes, disposées l'une par rapport à l'autre de telle manière que le produit à contrôler (9) puisse être déplacé le long de son axe longitudinal à travers exactement une (102.2) des au moins deux ouvertures de passage (102.1-102.4),
une installation de transport (91) pour le transport d'exactement un produit à contrôler (9) à travers exactement une (102.2) des au moins deux ouvertures de passage (102.1-102.4) qui a été choisie, et
un circuit de mesure capacitive (1), comprenant

au moins deux condensateurs de mesure (2.1-2.4) conçus chacun pour recevoir le produit à contrôler (9), chacun des au moins deux condensateurs de mesure (2.1-2.4) étant associé à l'une des au moins deux ouvertures de passage (102.1-102.4) de telle manière que sa capacité ($C_2$) puisse être influencée par un produit à contrôler (9) qui se trouve dans l'ouverture de passage (102.2) en question,
des moyens générateurs (4.1, 4.2) disposés pour appliquer au moins un signal électrique alternatif aux au moins deux condensateurs de mesure (2.1-2.4) et
au moins une ligne (5, 5.1-5.4) pour la sortie des signaux de sortie d'au moins un des condensateurs de mesure (2.1-2.4),

**caractérisé en ce que**
le circuit de mesure capacitif (1) présente des moyens de sélection (7, 8.1-8.8, 8.11-8.14) à actionnement électrique au moyen desquels un sous-ensemble propre (2.2) des l'ensemble de tous les condensateurs de mesure (2.1-2.4) peut être sélectionné de telle manière que seul le sous-ensemble (2.2) sélectionné participe à la mesure.

2. Dispositif selon la revendication 1, dans lequel chaque condensateur de mesure (2.1-2.4) est associé à un condensateur de compensation (3.1-3.4) pour compenser des influences qui perturbent la mesure, et chaque condensateur de mesure (2.2) forme avec le condensateur de compensation (3.2) qui lui est associé une paire de condensateurs (2.2, 3.2) montée en série.

3. Dispositif selon la revendication 2, dans lequel la ligne (5, 5.2) capte le signal de sortie entre le condensateur de mesure (2.2) et le condensateur de compensation (3.2) qui forment la paire de condensateurs (2.2, 3.2).

4. Dispositif selon l'une des revendications précédentes, dans lequel les moyens de sélection sont conformés comme des commutateurs (8.1-8.8) au moyen desquels une connexion électrique peut être établie et coupée individuellement entre les moyens générateurs (4.1, 4.2) et chacun des condensateurs de mesure (2.1-2.4).

5. Dispositif selon la revendication 4 et l'une des revendications 2 et 3, dans lequel les moyens générateurs (4.1, 4.2) sont disposés pour appliquer des signaux électriques alternatifs de même grandeur mais de phase opposée aux au moins deux condensateurs de mesure (2.1-2.4) ou aux au moins deux condensateurs de compensation (3.1-3.4) et les moyens de sélection sont conformés aussi bien comme des commutateurs (8.1-8.4) au moyen desquels une connexion électrique peut être établie et coupée individuellement entre les moyens générateurs (4.1) et chacun des condensateurs de mesure (2.1-2.4) que comme des commutateurs (8.5-8.8) au moyen desquels une connexion électrique peut être établie et coupée individuellement entre les moyens générateurs (4.2) et chacun des condensateurs de compensation (3.1-3.4).

6. Dispositif selon la revendication 4 ou 5, dans lequel chaque condensateur de mesure (2.2) est associé à au moins un commutateur (8.2, 8.6) et tous les condensateurs de mesure (2.1-2.4) peuvent être connectés à une ligne commune (5) pour la sortie des signaux de sortie.

7. Dispositif selon la revendication 6, dans lequel les moyens de sélection sont conformés comme des commutateurs (8.1-8.8) au moyen desquels une connexion électrique peut être établie et coupée individuellement entre les moyens générateurs (4.1, 4.2) et chacun des condensateurs de mesure (2.1-2.4) et comme des commutateurs (8.11-8.14) au moyen desquels une connexion électrique peut être établie et coupée individuellement entre chacun des condensateurs de mesure (2.1-2.4) et la ligne commune (5).

8. Dispositif selon l'une des revendications précédentes, dans lequel chaque condensateur de mesure (2.1-2.4) est associé à une ligne (5.1-5.4) individuelle pour la sortie de ses signaux de sortie et les moyens de sélection sont conformés comme un multiplexeur (7) au moyen duquel seuls les signaux provenant du sous-ensemble choisi (2.2) des condensateurs de mesure (2.1-2.4) peuvent être sélectionnés.

9. Procédé pour l'examen capacitif d'un produit à contrôler en forme de brin (9) dans un dispositif selon l'une des revendications précédentes, dans lequel le produit à contrôler (9) est introduit dans exactement une (102.2) des au moins deux ouvertures de passage (102.1-102.4) et déplacé à travers celle-ci le long de son axe longitudinal, tandis qu'aucun produit à contrôler ne se trouve dans les autres ouvertures de passage (102.1, 102.3, 102.4),

le produit à contrôler (9) influence une capacité ($C_2$) d'un condensateur de mesure (2.2) associé à l'ouverture de passage (102.2) en question,

au moins un signal électrique alternatif est appliqué au condensateur de mesure (2.2) associé à l'ouverture de passage (102.2) en question par les moyens générateurs (4.1, 4.2) et

des signaux de sortie d'au moins un des condensateurs de mesure (2.1-2.4) sont émis en sortie,

**caractérisé en ce que**

un sous-ensemble propre (2.2) de l'ensemble de tous les condensateurs de mesure (2.1-2.4) est sélectionné au moyen des moyens de sélection (7, 8.1-8.8) à actionnement électrique, de telle manière que seul le sous-ensemble (2.2) sélectionné participe à la mesure.

10. Procédé selon la revendication 9, dans lequel les influences perturbant la mesure sont compensées par des condensateurs de compensation (3.1-3.4), dont chacun est associé à un condensateur de mesure (2.1-2.4).

11. Procédé selon la revendication 9 ou 10 dans lequel, en vue de la sélection, l'au moins un signal électrique alternatif est appliqué uniquement au sous-ensemble sélectionné (2.2) tandis que l'ensemble (2.1, 2.3, 2.4) complémentaire du sous-ensemble sélectionné (2.2) est désactivé.

12. Procédé selon la revendication 11, dans lequel des signaux de sortie de tous les condensateurs de mesure (2.1-2.4) sont émis ensemble en sortie.

13. Procédé selon l'une des revendications 9 à 11, dans lequel seuls les signaux de sortie du sous-ensemble sélectionné (2.2) sont émis en sortie, tandis que les signaux de sortie de l'ensemble (2.1, 2.3, 2.4) complémentaire du sous-ensemble sélectionné (2.2) sont rejetés.

14. Procédé selon les revendications 11 et 13, dans lequel, en vue de la sélection, l'au moins un signal électrique alternatif est appliqué uniquement au sous-ensemble sélectionné (2.2) tandis que l'ensemble (2.1, 2.3, 2.4) complémentaire du sous-ensemble sélectionné (2.2) est désactivé, et seuls les signaux de sortie du sous-ensemble sélectionné (2.2) sont émis en sortie, tandis que les signaux de sortie de l'ensemble (2.1, 2.3, 2.4) complémentaire du sous-ensemble sélectionné (2.2) sont rejetés.

15. Procédé selon la revendication 13, dans lequel l'au moins un signal électrique alternatif est appliqué à tous les condensateurs de mesure (2.1-2.4) et seuls les signaux de sortie du sous-ensemble sélectionné (2.2) sont émis en sortie.

Fig. 1

Fig. 2    STAND DER TECHNIK

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0924518 A1
- WO 2005033697 A1
- US 3731069 A
- US 6369588 B1
- WO 2008128363 A1
- WO 2010043064 A1
- WO 2010043063 A1
- WO 2010043065 A1